# EUROPEAN PATENT SPECIFICATION

(11) **EP 3 820 466 B1**
(45) Date of publication and mention of the grant of the patent: **07.09.2022**
(21) Application number: 19749778.7
(22) Date of filing: 05.07.2019
(51) Int. Cl.: A61K 31/353, A61K 31/716, A61K 31/80, A61K 36/45, A61K 38/40, A61J 3/00, A61K 9/00, A61K 9/107, A61K 9/14

(54) **NUTRITIONAL SUPPLEMENT, SUITABLE FOR ORAL ADMINISTRATION, COMPRISING LACTOFERRIN, XYLOGLUCAN, PROANTHOCYANIDINS AND SIMETHICONE FOR THE JOINT PREVENTION OF GASTROINTESTINAL AND URINARY TRACT INFECTIONS**
NAHRUNGSERGÄNZUNG ZUR ORALEN VERABREICHUNG MIT LACTOFERRIN, XYLOGLUCAN, PROANTHOCYANIDINEN UND SIMETHICON ZUR GEMEINSAMEN VORBEUGUNG VON INFEKTIONEN DES MAGEN-DARM- UND DES HARNTRAKTES
SUPPLÉMENT NUTRITIONNEL, APPROPRIÉ POUR UNE ADMINISTRATION ORALE, COMPRENANT DE LA LACTOFERRINE, DU XYLOGLUCANE, DES PROANTHOCYANIDINES ET DE LA SIMÉTHICONE, POUR LA PRÉVENTION CONJOINTE D'INFECTIONS GASTRO-INTESTINALES ET DU TRACTUS URINAIRE

(30) Priority: 09.07.2018 GR 20180100307
(43) Date of publication of application: 19.05.2021
(73) Proprietor: Tseti, Ioulia, 145 61 Kifissia Attikis (GR)
(72) Inventor: Tseti, Ioulia, 145 61 Kifissia Attikis (GR)
(74) Representative: Wibbelmann, Jobst
(86) International application number: PCT/GR2019/000049
(87) International publication number: WO 2020/012203

(56) References cited:
- EP-A1- 0 904 784
- EP-A1- 2 674 371
- US-A- 5 980 944
- US-A1- 2013 316 025
- US-A1- 2015 094 266
- US-B1- 6 231 866

## Description

The present invention relates to a nutritional supplement, as a single dose oral suspension, comprising lactoferrin, xyloglucan, proanthocyanidins and simethicone as active ingredients, that is helpful in the joint prevention of gastrointestinal and urinary tract infections. The nutritional supplement of the invention is characterized in that it is prepared just before its use and it can be consumed directly from its container, which consists of a vial containing a flavored simethicone liquid composition, further provided with a closing storage cap containing a composition in solid powdered form comprising lactoferrin, xyloglucan and cranberry extract, standardized in proanthocyanidines. The closing storage cap of the device of the invention ensures that the solid powdered composition of lactoferrin, xyloglucan and cranberry extract is stored tightly separated from the liquid composition of simethicone stored in the vial of the device, thus remaining intact for prolonged time. The solid contents of the closing storage cap are transferred to the liquid composition of the vial by an immediate procedure, just before the time of use, forming a single-dose oral flavored suspension which can be used directly from its container.

Lactoferrin is a mammalian cationic non-haem glycoprotein of average molecular weight 80 kDa and is present in many body secretions including saliva, tears and breast milk. Lactoferrin is also present in cow's milk. Lactoferrin has antiviral, antibacterial and antifungal properties as well as being an antioxidant. It binds iron and helps to transport it more efficiently around the body, while at the same time, minimizes the free radical damage that unbound iron can cause. Furthermore, lactoferrin inhibits the survival, growth and spread of bacteria, by binding to the outer membrane proteins in Gram-negative bacteria and to teichoic acid in Gram-positive bacteria, thus, causing disruption of the bacterial membrane, leading to cell lysis. Finally, it encourages the growth of good gut flora within the stomach. Lactoferrin is the second most abundant protein in breast milk and because of all above-mentioned beneficial effects, it is considered responsible for conferring immunity on newborns in the first weeks of their life. Lactoferrin is a stable protein that retains its physicochemical characteristics under gastric pH conditions, and in most cases it is bioactive even after digestion, thus, the incorporation of lactoferrin into diets facilitates its administration. Commercially available nutritional supplements, containing lactoferrin, commonly provide between 250-750 mg lactoferrin per day. Several studies in humans suggest that the consumption of 200 mg lactoferrin on a daily base is sufficient to support immune function. European Union guidelines accept a daily dosage of up to 3 g in case of specific medical purposes. In general, lactoferrin appears to be a safe nutritional supplement with no known adverse effects. Document EP 2 968 305 relates to methods of reducing the duration of diarrhea symptoms and reducing gastrointestinal irritation in children by providing an oral electrolyte solution comprising lactoferrin.

Xyloglucans are molecules with a linear [1,4]-β-glucan backbone, which is the same as cellulose, with short [1,6]-α-xylose side chains that are partially substituted by other sugars such as arabinose and fucose. Xyloglucan has an average molecular weight of 55 kDa and forms a highly viscous solution in water with a broad pH tolerance and high thermal stability. Seeds of tamarind (Tamarindus indica), a tropical tree originating from East Africa, represent the main source of xyloglucan. Tamarind seeds consist of about 65-72 % polysaccharides, 15-23% protein and 4-7% lipids while they contain no free sugars. Several studies suggest that xyloglucan, either alone or in combination with oral rehydration solutions, is particularly effective in the prevention of diarrhea and urinary tract infections. Xyloglucan is considered as a "mucosal protector" and its mechanism is based on a film formation in the intestinal and uroepithelial mucosa which reduces the permeability of the tight junctions of the mucosa, therefore preventing the entry of the pathogens responsible for acute intestinal and urinary tract infections. The film-forming effect is not affected by variations in pH value. *In vitro* studies have also proved the protective effect of xyloglucan against *Escherichia coli* invasion of intestinal and uroepithelial cells (Pique, N. *et al*.).

Cranberry fruits mainly consist of phenolic compounds including flavonols, flavan-3-ols, anthocyanins, proanthocyanidins (PACs), catechins, phenolic acid derivatives, and triterpenoid analogues. Cranberry fruits contain a great percentage of A-type PACs, where the two flavan-3-ol building blocks are condensed with an ether-type bond between C2 of the upper unit and the hydroxyl group at C7 of the lower unit. *In vitro* studies showed that A-type PACs from cranberries prevent the adherence of *Escherichia coli* fimbriae to uroepithelial cells, thereby blocking *Escherichia coli* from sticking to the bladder wall (Vasileiou I. *et al*.). However, due to the uncertain and limited availability of PACs, especially in commercially available cranberry juices, it is assumed that the concentrations needed for this mechanical mode of action are likely not be reached in the bladder.

Simethicone is a mixture of repeating linear polydimethylsiloxane units of the formula [-(CH₃)₂SiO-]*ₙ*, where 90<n<410, ending to a trimethylsiloxy-group of the formula [(CH₃)₃SiO-], and stabilized with silicon dioxide. It has a minimum molecular weight of 6800 and contains polydimethylsiloxane groups in a percentage between 90.5 % and 99.0 % and silicon dioxide between 4.0 % and 7.0 %. Simethicone is able to decrease the surface tension of a liquid, therefore, can prevent bubble formation and gas retention in the foam and can help to eliminate flatulence (Brecevic L. *et al.).* Taking advantage of these properties, simethicone is used as an anti-foaming and anti-flatulent agent in order to relieve painful pressure caused by excess gas in the stomach and intestines.

Acute diarrhea with gas-related abdominal discomfort is a common, usually self-limited disorder which may be caused by a temporary problem, like a bacterial, viral or parasitic infection, side effects from drugs, especially antibiotics, food intolerances and allergies, or a chronic problem, like an intestinal disease. In most cases, diarrhea is self-treated after a few days, however, serious morbidity effects appear due to repeated attacks of diarrhea and are associated with electrolyte imbalances, dehydration and defective immune system responses. Orally consumed rehydration solutions, based on glucose and sodium mixtures, usually replace lost fluids and electrolytes in the human body, while in more serious incidents, a proper medication is necessary.

Urinary tract infections (UTIs) are one of the most widespread bacterial diseases. It affects more than 30 % of women globally during their lifetime, resulting in high dysphoria, illness and multiple antibiotic treatments. The main pathogen involved in these infections is Gram-negative bacteria, primarily *Escherichia coli,* which can colonize the genital area, including the periurethral zone, and can reach the urethra and even the bladder.

The invention relates to a nutritional supplement comprising the unique combination of lactoferrin, xyloglucan, proanthocyanidines and simethicone in the form of a single dose oral suspension with pleasant flavor, which is prepared just before its use and helps in the prevention of both intestinal and urinary tract infections.

There is not a single reference in the prior art that concerns a nutritional supplement comprising the unique combination of lactoferrin, xyloglucan, cranberry extract standardized in proanthocyanidines and simethicone for the prevention of both intestinal and urinary tract infections. Any prior attempts to prepare a dietary supplement, in the form of a single dose oral solution or suspension containing lactoferrin, xyloglucan, proanthocyanidins and simethicone, were unsuccessful. Most of the proposed solutions or suspensions were rapidly degraded due to aggregation of their components, developing a colloidal mass. This problem was resolved when it was surprisingly found that the mixture of lactoferrin, xyloglucan and cranberry extract standardized in proanthocyanidins remains stable when stored in a solid form and tightly separated from the simethicone suspension. In addition, it was found that the solid mixture of lactoferrin, xyloglucan and cranberry extract standardized in proanthocyanidins is readily mixed with a flavored suspension of simethicone, creating a pleasant preparation ready for immediate intake.

The air-tight separation of the solid mixture comprising lactoferrin, xyloglucan, and cranberry extract standardized in proanthocyanidines, from simethicone suspension is achieved by using a container based on a closing storage cap-vial system, in which the closing storage cap contains the composition of lactoferrin, xyloglucan and cranberry extract in powder form while the vial contains the simethicone suspension. The closing storage cap which contains the solid powdered composition is sealed tightly on the vial containing simethicone suspension. The nutritional supplement of the invention is prepared just prior its use by an immediate procedure and it can be consumed directly from the vial.

There is need for natural ways to help the prevention of gastrointestinal and urinary tract infections in humans and this is solved by proposing a nutritional composition comprising the combination of an antimicrobial protein, as lactoferrin, a "mucosal protector" as xyloglucan and the standardized in proanthocyanidins cranberry extract and an anti-gas agent as simethicone. The nutritional supplement of the present invention contains all the above active ingredients in a prophylactic effective dose, in particular, in a concentration similar to the corresponding one having a beneficial effect in the immune system, thus providing a joint and synergistic effect on the prevention of gastrointestinal and urinary tract infections. The effective dose can be readily determined by a person skilled in the art.

The present invention takes advantage of the synergistic effect of lactoferrin and xyloglucan against diarrhea symptoms and the synergistic effect of xyloglucan and proanthocyanidins contained in cranberry extract against urinary tract infections and suggests a new composition comprising lactoferrin, xyloglucan and cranberry extract, further enriched with simethicone, suitably presenting a joint beneficial effect against gastrointestinal and urinary tract disorders.

It is well known that lactoferrin is very sensitive to chemical and microbial degradation when it is stored in solution at ambient temperature. Thus, the long-term storage of lactoferrin in a stable solid form and the ability to transfer it into solution, just before the time of use, is one of the problems solved by the present invention.

Another problem solved by the present invention is to provide stable and consistent doses of proanthocyanidins by using a cranberry extract that is standardized in proanthocyanidins. This verifies that definite and sufficient quantities of proanthocyanidins are reached in the bladder, something that it could not be achieved by using commercially available cranberry juices.

The nutritional supplement of the present invention based on the unique combination of lactoferrin, xyloglucan, proanthocyanidins and simethicone, which presents a synergistic effect in the prevention of intestinal and urinary tract infections, is stored in a closing storage cap-vial system as those disclosed in the documents EP 2 922 767, EP 2 674 371 and EP 1 623 932. Although the device of the present invention is not limited by the systems described in the aforementioned documents, however, the closing storage cap-vial system described in EP 1 623 932 is one of the preferred ones.

In a preferred embodiment, the closing storage cap of the container of the nutritional supplement of the present invention contains a solid powdered composition comprising lactoferrin, xyloglucan and cranberry extract, further comprising a diluent and a lubricant, both aiding to the binding of the constituents and to enhance the rheological properties of the final blend.

In a preferred embodiment, lactoferrin used in the solid powdered composition of the nutritional supplement of the present invention, is a non-human lactoferrin, preferably is bovine lactoferrin isolated from cow's milk. Xyloglucan used in the present invention is isolated from seeds of Tamarind. Cranberry extract used in the present invention is standardized to contain proanthocyanidins in the range from 25% to 60%. All active ingredients of the solid composition come from commercially available products. According to the present invention the % weight ratio of lactoferrin:xyloglucan:cranberry extract:simethicone in the final suspension of the nutritional supplement of the present invention is from 1:1:1:1 to 5:2:2:2, more preferably is 3:1:1:1.2.

In a preferred embodiment, the solid powdered composition in the closing storage cap comprises from 250 mg to 750 mg lactoferrin, which corresponds to a percentage from 2.5% to 7.5% of a dosage unit of the nutritional supplement of the present invention.

In a preferred embodiment, the solid powdered composition in the closing storage cap comprises from 50 mg to 250 mg xyloglucan, which corresponds to a percentage from 0.5% to 2.5% of a dosage unit of the nutritional supplement of the present invention.

In a preferred embodiment, the solid powdered composition in the closing storage cap comprises from 50 mg to 250 mg cranberry extract, which corresponds to a percentage from 0.5% to 2.5% of a dosage unit of the nutritional supplement of the present invention. According to present invention the cranberry extract is standardized in proanthocyanidins, more preferably in A-type proanthocyanidins.

According to present invention, the vial of the container contains a liquid composition in the form of a flavored emulsion. This emulsion comprises simethicone in the form of 30% emulsion, further comprises appropriate excipients such as diluents, viscosity and pH regulators, flavor enhancers, sweeteners and preservatives.

Simethicone's role in the composition is two-fold: it acts as an emulsifier aiding to the efficient mixing of all active ingredients in the final composition and at the same time as an anti-gas agent in the stomach and intestines.

In a preferred embodiment, the suspension in the vial comprises from 200 mg to 600 mg of a 30% simethicone emulsion which corresponds to a percentage from 2.0% to 6.0% of a dosage unit of the nutritional supplement of the present invention.

In a preferred embodiment, the suspension in the vial comprises purified water as a solvent in an amount corresponding from 55% to 75% of a dosage unit of the nutritional supplement of the present invention.

In a preferred embodiment, the suspension in the vial comprises a diluent among mannitol, maltitol, sorbitol and xylitol, in an amount corresponding from 1500 mg to 4000 mg per dosage unit of the nutritional supplement of the present invention.

In a preferred embodiment, the suspension in the vial comprises one or more viscocity regulators among microcrystalline cellulose, carboxymethyl cellulose and hydroxyethyl cellulose, in an amount corresponding from 15 mg to 55 mg per dosage unit of the nutritional supplement of the present invention

In a preferred embodiment, the suspension in the vial comprises one or more pH regulators among acetic acid, citric acid, fumaric acid, hydrochloric acid, sorbic acid, tartaric acid, and their salts or sodium hydroxide, sodium bicarbonate, sodium carbonate and combinations thereof.

In a preferred embodiment, the suspension in the vial comprises a flavor enhancer, among lemon flavor, apricot flavor, cherry flavor, strawberry flavor, raspberry flavor, caramel flavor, honey flavor, and coffee flavor.

In a preferred embodiment, the suspension in the vial comprises a sweetener, among glucose, sucrose, fructose, sodium saccharinate and sodium cyclamate.

In a preferred embodiment, the suspension in the vial comprises a preservative among benzoic acid, sodium benzoate, benzyl alcohol, methyl p-hydroxybenzoate and propyl p-hydroxybenzoate and combinations thereof.

In a preferred embodiment the nutritional supplement in the form of a suspension prepared from the mixing of the solid powdered composition in the closing storage cap with the suspension in the vial is considered as a dosage unit and has a final volume of 10 mL.

In a preferred embodiment, the nutritional supplement in the form of a suspension prepared from the mixing of the solid powdered composition in the closing storage cap with the suspension in the vial is administered once a day.

According to the present invention, the solid composition of lactoferrin, xyloglucan and cranberry extract, stoed in the closing storage cap of the container of the nutritional supplement of the present invention, is prepared by mixing the appropriate pre-sieved quantities of the active ingredients in the presence of a diluent aiding in the efficient binding of the three components and a lubricant aiding in the enhancement of the rheological properties of the final blend. The flow characteristics of the final powdered blend are evaluated by measuring the bulk density and the tapped density of the powder and calculating the Hausner ratio which should ensure a good flow character. According to the present invention, the flavored suspension contained in the vial of the container of the nutritional supplement of the present invention, is prepared by dissolving the excipients such as diluents, viscosity regulators, flavor enhancers, sweeteners and preservatives in purified water, following by addition of simethicone 30% emulsion and finally by regulating the pH value of the final solution before bringing it to the final volume with purified water.

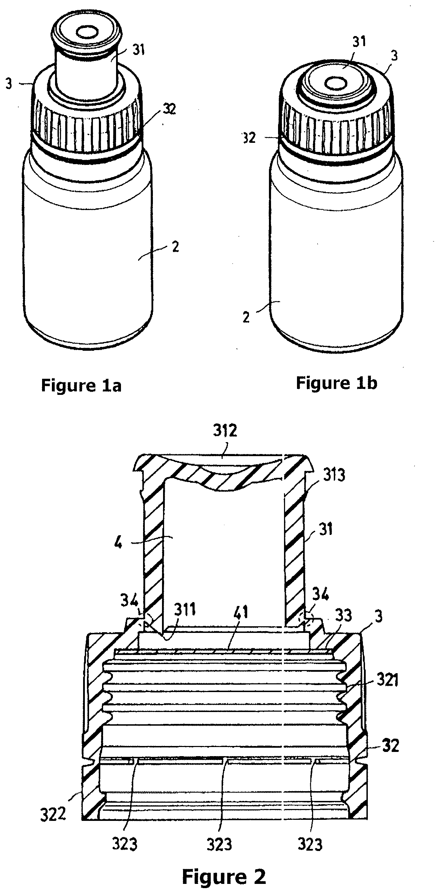

The characteristic features of the container of the nutritional supplement of the present invention based on the closing storage cap-vial system are better understood with reference to the attached Figures 1 and 2, as these have been already described in EP 1 623 932 B1. Figure 1a shows a view of the system closing storage cap (3) and vial (2) before mixing the compositions contained in the vial 2 and in the closing storage cap 3, respectively; Figure 1b shows a view of the system closing storage cap and vial after mixing the substances contained in the vial 2 and in the closing storage cap 3, respectively. Accordingly, Figure 2 shows a section of the closing storage cap 3 where the solid composition of the nutritional supplement according to the present invention, is stored.

In a preferred embodiment, the container of the nutritional supplement of the present invention comprising a closing storage cap suitable to seal a cylindrical vial (2), said cap comprising: a) a hollow capsule (3) provided with fixed attachment means to said vial, said capsule comprising a reservoir (4) in which the solid powdered composition consisting of lactoferrin, xyloglucan and cranberry extract is contained, in order to be kept separate from the flavored liquid composition of simethicone contained in said cylindrical vial (2) until the moment of use; b) breaking means of the base of said reservoir that can be actuated with pressure from the top of said capsule, said breaking means comprise at least one fracture point (311) suitable for penetrating into a membrane (41) causing it to break and causing the first substance contained in the reservoir (4) to fall by gravity into the cylindrical vial (2). The capsule (3), the reservoir (4) and the breaking means are made in a single body, said capsule comprises an upper portion (31) in which said reservoir (4) is integrated and a lower portion (32), having a greater diameter than the diameter of the upper portion, provided with said fixed attachment means to the vial, furthermore, said capsule comprises an annular weakening sector (34) laying between the upper portion and the lower portion of the capsule, which fractures by exerting pressure on the top of the capsule, causing the upper portion of the capsule itself to insert into the lower portion, with simultaneous breakage of the membrane (41) placed to the edge point (33), which also specifies the joint of the upper portion with the lower portion of the capsule, through the fracture point (311), which is arranged along said annular weakening sector (34).

The invention is further described with the following non-limiting examples:
Example 1. Powdered composition comprising lactoferrin, xyloglucan and cranberry extract

Each one of the active ingredients in solid form, namely, lactoferrin, xyloglucan and cranberry extract, passed through a 150 mesh sieve to ensure that the particle size of each active ingredient is less than 105 µm. Subsequently, the sieved active ingredients are placed in a drum mixer. Sorbitol and colloidal silicon dioxide are added in the mixer and the solid mixture homogenized for 45 min at 40 rpm (rounds per minute). The flow character of the final blend is evaluated by the Hausner Ratio. Bulk and tapped density of samples collected from five different places of the drum mixer measured and the results summarized in Table 1.

**Table 1. Powder flow analysis of the composition comprising lactoferrin, xyloglucan and cranberry extract.**

| **Sample** | **Bulk Density (g/mL)** | **Tapped Density (g/mL)** | **Hausner Ratio** |
|---|---|---|---|
| 1 | 0.575 | 0.487 | 1.18 |
| 2 | 0.558 | 0.483 | 1.15 |
| 3 | 0.572 | 0.485 | 1.18 |
| 4 | 0.582 | 0.489 | 1.19 |
| 5 | 0.590 | 0.488 | 1.21 |

Based on the results obtained, the samples of the powdered composition comprising lactoferrin, xyloglucan and cranberry extract, are considered as "good" to "very good" in terms of their flow based on Hausner Ratio values. This ensures the effective flow of the solid composition located in the closing storage cap into the vial of the container of the nutritional supplement of the invention, at the time of use. The final powdered blend comprising lactoferrin, xyloglucan and cranberry extract, showing very good flowing characteristics, is placed in the closing storage caps and sealed in the presence of an inert gas.

In a preferred embodiment, the solid powdered composition contained in a closing storage cap of the device of the nutritional supplement of the present invention contains the following ingredients:

| **Ingredients in closing storage cap** | **Quantity (mg) per dosage unit** |
|---|---|
| Lactoferrin | 300.0 |
| Xyloglucan | 100.0 |
| Cranberry extract (standardized in 40% PACs) | 100.0 (corresponds to 40 mg PACs) |
| Sorbitol | 50.0 |
| Silicon dioxide anhydrous | 4.0 |

### Example 2. Preparation of the suspension filled in the vial

Purified water, equal to 70% of the total amount used in the liquid composition placed in a stainless steel reactor. The whole amount of a mixture of microcrystalline cellulose and carboxymethylcellulose (as the commercially available AVICEL^{®} RC-591) placed in the reactor and mixture stirred at room temperature for 15 min. Subsequently, and under continuous stirring, the following ingredients were added in the reactor, in the specified order: maltitol liquid, citric acid, trisodium citrate, sodium benzoate, raspberry flavor and sodium saccharine. The mixture was further stirred for 45 min till to obtain a homogeneous solution. Then, hydroxyethylcellulose was added and the mixture stirred for another 15 min. Finally, Simethicone emulsion 30% was added under stirring and the pH of the liquid composition adjusted to 3.8 with HCI 0.1 N and then set up in the final volume with purified water. The final liquid composition is placed in the vials of the device of the invention, which then were tightly sealed with the closing storage caps containing the solid powdered composition of lactoferrin, xyloglucan and cranberry extract.

In a preferred embodiment, the suspension contained in a vial contains the following ingredients:

| **Ingredients in liquid form (final volume 10 m)** | **Quantity (mg) per vial** |
|---|---|
| Simethicone emulsion 30% | 400.0 |
| Microcrystalline Cellulose + carboxymethylcellulose (AVICEL RC-591) | 10.0 |
| Hydroxyethylcellulose | 26.0 |
| Citric Acid Anhydrous | 12.0 |
| Trisodium Citrate | 5.0 |
| Maltitol Liquid (Lycasin) | 2000.0 |
| Sodium Benzoate | 25.0 |
| Raspberry flavor | 25.0 |
| Sodium Saccharine | 5.0 |
| Water Purified | q.s. 10 mL |

### Example 3. Description of administration.

The nutritional supplement of the present invention, characterized in that is placed in a closing storage cap-vial system as the one disclosed in EP 1 623 932, where the closing storage cap contains a solid powdered composition comprising lactoferrin, xyloglucan and cranberry extract and the vial contains a flavored liquid composition of simethicone, operates in the following way. Upon the time of use, the consumer exerts pressure with a finger to the upper section of the closing storage cap-vial system (through the dome 312), where, the annular weakening sector 34 fractures and the upper portion inserts inside the lower portion of the closing cap. At this point, the fracture point 311 penetrates into the membrane 41 and breaks it, thus causing the powdered composition of lactoferrin, xyloglucan and cranberry extract, contained in the reservoir 4 of the closing storage cap, to fall by gravity into the cylindrical vial 2, consequently, coming in contact with the liquid simethicone based composition contained therein. Once the mixing in the vial 2 between the two compositions has occurred, the closing storage cap can be unscrewed and removed away. The liquid suspension created in the vial, comprising of lactoferrin, xyloglucan, cranberry extract and simethicone presents an acceptable for oral administration pH value of 4.3, has a pleasant taste and can be consumed orally as a single dosage unit.

### References Cited

1. EP 2 968 305: Oral electrolyte solution containing lactoferrin and uses thereof.
2. Pique, N.; Gomez-Guillen, M.C.; Montero, M.P. Xyloglucan, a Plant Polymer with Barrier Protective Properties over the Mucous Membranes: An Overview. Int. J. Mol. Sci. 2018, 19, 673.
3. Vasileiou, I.; Katsargyris, A.; Theocharis, S.; Giaginis, C. Current clinical status on the preventive effects of cranberry consumption against urinary tract infections. Nutrition Research 2013, 33, 595.
4. Brecevic, L.; Bosan-Kilibarda, I.; Strajnar, F. Mechanism of Antifoaming Action of Simethicone. J. Appl. Toxicol. 1994, 14, 207.
5. EP 2 922 767: Device for the fluid-tight conservation of a substance to be mixed to another substance contained in a container.
6. EP 2 674 371: Bottle cap for the conservation of substances to be kept separate until their use.
7. EP 1 623 932: Closing device with chamber for a container of substances to be kept separate until dispensing.

## Claims

1. A nutritional supplement, in the form of a single-dose oral suspension, comprising lactoferrin, xyloglucan, cranberry extract and simethicone, **characterized in that** lactoferrin, xyloglucan and cranberry extract are stored in powdered form tightly separated from simethicone, which is kept as emulsion in a closing storage cap-vial container system, further **characterized in that** the solid composition comprising lactoferrin, xyloglucan and cranberry extract is placed in the closing storage cap of the container and is immediately mixed just prior its use with the simethicone liquid composition contained in the vial of the container, thus forming an oral suspension suitable for immediate use from the vial.

2. The nutritional supplement according to claim 1 wherein the % weight ratio between lactoferrin:xyloglucan:cranberry extract:simethicone in the final suspension is from 1:1:1:1 to 5:2:2:2.

3. The nutritional supplement according to claims 1 and 2 wherein the cranberry extract is standardized between 25 % and 60% in proanthocyanidins.

## Patentansprüche

1. Nahrungsergänzungsmittel in Form einer oralen Einzeldosis-Suspension, umfassend Lactoferrin, Xyloglucan, Cranberry-Extrakt und Simethicon, **dadurch gekennzeichnet, dass** Lactoferrin, Xyloglucan und Cranberry-Extrakt in pulverisierter Form dicht getrennt von Simethicon aufbewahrt werden, das als Emulsion in einem verschließbaren Aufbewahrungskappen-Fläschchen-Behältersystem gehalten wird, **dadurch gekennzeichnet, dass** die feste Zusammensetzung, die Lactoferrin, Xyloglucan und Cranberry-Extrakt umfasst, in die verschließbare Aufbewahrungskappe des Behälters gegeben wird und unmittelbar vor ihrer Verwendung mit der flüssigen Simethicon-Zusammensetzung, die in dem Fläschchen des Behälters enthalten ist, gemischt wird, wodurch eine orale Suspension gebildet wird, die zur sofortigen Verwendung aus dem Fläschchen geeignet ist.

2. Nahrungsergänzungsmittel nach Anspruch 1, wobei das prozentuale Gewichtsverhältnis zwischen Lactoferrin:Xyloglucan:Cranberry-Extrakt:Simethicon in der endgültigen Suspension von 1:1:1:1 bis 5:2:2:2 beträgt.

3. Nahrungsergänzungsmittel nach den Ansprüchen 1 und 2, wobei der Cranberry-Extrakt zwischen 25 % und 60 % an Proanthocyanidinen standardisiert ist.

## Revendications

1. Complément nutritionnel, sous la forme d'une suspension buvable unidose, comprenant de la lactoferrine, du xyloglucane, de l'extrait de canneberge et de la siméthicone, **caractérisée en ce que** la lactoferrine, le xyloglucane et l'extrait de canneberge sont stockés sous forme de poudre étroitement séparés de la siméthicone, qui est conservée sous forme d'émulsion dans un système de récipient capuchon de stockage à fermeture-flacon, **caractérisée en outre en ce que** la composition solide comprenant de la lactoferrine, du xyloglucane et de l'extrait de canneberge est placée dans le capuchon de stockage à fermeture du récipient et est immédiatement mélangée juste avant son utilisation avec la composition liquide de siméthicone contenue dans le flacon du récipient, formant ainsi une suspension buvable appropriée pour une utilisation immédiatement à partir du flacon.

2. Complément nutritionnel selon la revendication 1, dans lequel le rapport pondéral en % entre lactoferrine:xyloglucane:extrait de canneberge:siméthicone dans la suspension finale est de 1:1:1:1 à 5:2:2:2.

3. Complément nutritionnel selon les revendications 1 et 2 dans lequel l'extrait de canneberge est standardisé entre 25 % et 60 % en proanthocyanidines.
